(19) Europäisches Patentamt
European Patent Office
Office européen des brevets

(11) Numéro de publication: **0 233 100 B1**

# (12) FASCICULE DE BREVET EUROPEEN

(45) Date de publication de fascicule du brevet: **07.08.91**

(51) Int. Cl.$^5$: **A61K 9/50**

(21) Numéro de dépôt: **87400057.3**

(22) Date de dépôt: **13.01.87**

Le dossier contient des informations techniques présentées postérieurement au dépôt de la demande et ne figurant pas dans le présent fascicule.

## (54) Composition pharmaceutique,contenant un anesthésique local et/ou un analgésique central encapsulé dans des multilamellaires liposomes.

(30) Priorité: **14.01.86 FR 8600434**

(43) Date de publication de la demande: **19.08.87 Bulletin 87/34**

(45) Mention de la délivrance du brevet: **07.08.91 Bulletin 91/32**

(84) Etats contractants désignés: **AT BE CH DE ES FR GB GR IT LI LU NL SE**

(56) Documents cités:
**EP-A- 0 152 379**

**CHEMICAL ABSTRACTS, vol. 94, 1981, page 66, résumé no. 58303h, Columbus, Ohio, US; T. OKANO et al.: "Duration of the local anesthetic action of dibucaine by liposomes and its mechanism", & YAKUGAKU ZASSHI 1980, 100(11), 1097-103**

(73) Titulaire: **l'Université Libre de Bruxelles Centre de Recherches Industrielles Avenue F.D. Roosevelt 50 B-1080 Bruxelles(BE)**

(72) Inventeur: **Legros, Franz 313 Chaussée de Boondael B-1050 Bruxelles(BE)**
Inventeur: **Ruysschaert, Jean-Marie Avenue Perce-Neige 7 B-1640 Rhode-Saint-Genese(BE)**

(74) Mandataire: **Van Malderen, Michel et al p.a. Office van Malderen avenue J.S. Bach 22/43 B-1080 Bruxelles(BE)**

EP 0 233 100 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

CHEMICAL ABSTRACTS, vol. 96, 1982, page 136, résumé no. 116500z, Columbus, Ohio, US; M.F. KHAN: "Enhanced analgesic activity of liposomised Met-enkephalin analog", & INDIAN J. BIOCHEM. BIOPHYS. 1981, 18(6), 440-1

CHEMICAL ABSTRACTS, vol. 86, 1977, page 26, résumé no. 165153r, Columbus, Ohio, US; P.L. YEAGLE et al.: "Molecular dynamics of the local anesthetic tetracaine in phospholipid vesicles", & BIOCHIM. BIOPHYS. ACTA 1977, 465(2), 173-8

BIOCHIMICA ET BIOPHYSICA ACTA, vol. 799, 1984, pages 195-198, Elsevier Science Publishers B.V.; N. KRAUS-FRIEDMANN et al.: "Liposome encapsulated tetracaine lowers blood glucose"

## Description

La présente invention concerne des compositions pharmaceutiques pour l'anesthésie, et principalement l'anesthésie péridurale. L'anesthésie péridurale est une anesthésie loco-régionale de conduction réalisée par l'injection d'un anesthésique local dans l'espace péridural.

Cet espace est situé entre la dure-mère et les parois ostéo-ligamentaires du canal rachidien. Il est fermé vers le haut par la dure-mère qui adhère intimement au pourtour du trou occipital. Vers le bas, la dure-mère se termine au niveau de la deuxième vertèbre sacrée. A ce niveau, se détache le film terminal qui va adhérer au coccyx. L'espace péridural s'étend donc à partir du trou occipital jusqu'à la membrane sacrocossygienne. Il contient un tissu cellulo-adipeux lâche, les racines de nerfs rachidiens, des artères spinales, des plexus veineux rachidiens, des canaux lymphatiques.

Le principe de l'analgésie péridurale est connu depuis très longtemps puisque cette technique est presque aussi ancienne que l'anesthésie locale à la cocaïne.

Les anesthésiques locaux agissent sur la fibre nerveuse en interférant avec les processus d'excitation-conduction. Mais les fibres nerveuses sont inégalement sensibles à l'action des anesthésiques locaux. Ce sont d'abord les fibres neuro-végétatives qui sont touchées (blocage sympathique), puis les fibres sensitives (disparaissent dans l'ordre : les sensations douloureuses, thermiques, tactiles) ; les fibres motrices sont touchées les dernières.

De nombreuses substances présentent des propriétés anesthésiques locales, mais ne sont pas utilisées parce qu'elles ne possèdent pas toutes les qualités d'un bon anesthésique local.

Et en particulier, parmi les anesthésiques dérivés de la cocaïne, des variations dans l'intensité de l'action existent selon les produits. Ainsi, par exemple, la bupivacaïne atteint peu ou pas les fibres motrices.

Remédier à l'action insuffisamment puissante des anesthésiques par une augmentation des doses conduirait à une toxicité certaine. En effet, un pourcentage non négligeable de l'anesthésique, même administré par voie péridurale, emprunte la voie sanguine et/ou la voie rachidienne puis céphalorachidienne, pour se diriger dans divers organes en particulier aux niveaux du coeur et du foie et/ou du système nerveux central.

Ainsi, si pour pallier à une action trop faible ou de durée trop courte, l'on injectait une plus forte dose d'anesthésique, celle-ci risquerait de produire des effets toxiques très graves voire irrémédiables, allant, par exemple, d'une chute de la pression artérielle, à des troubles du rythme cardiaque, voire à un arrêt cardiaque.

La présente invention propose une solution pour remédier aux inconvénients précités.

En effet, la présente invention concerne des compositions pharmaceutiques, caractérisées en ce qu'elles contiennent un anesthésique local et/ou un analgésique central, comportant une partie lipophile, encapsulé dans des liposomes.

En effet, la longue durée d'action des anesthésiques lipophiles administrés dans l'espace péridural sous une forme hydrosoluble a été attribuée essentiellement a leur captage et séquestration par le tissu adipeux.

Le document EP-A-0 152 379 décrit l'encapsulation d'un grand nombre de composés parmi lesquels figurent des anesthésiques et des analgésiques dans des liposomes unilamellaires, sans mentionner l'utilisation des liposomes multilamellaires.

La publication du Chemical Abstracts 96:116500z décrit l'utilisation analgésique de met-enképhaline encapsulés dans des liposomes par administration intracérébroventriculaire.

La publication du Chemical Abstracts 86:165153 décrit l'incorporation de tétracaïne dans des vésicules lipidiques de phosphatydilcholine contenant 30% de cholestérol.

La publication du Chemical Abstracts 94:5830h décrit l'action anesthésique de dibucaïne incorporée dans des liposomes.

La publication du Bioch. Bioph. Acta, 799 de 1984 décrit l'action antidiabétique de tétracaïne incorporée dans des liposomes de composition particulière.

Or, les liposomes sont des vésicules constituées d'une double couche de phospholipides dans une solution aqueuse, avec leurs parties hydrophiles en contact avec l'eau à l'extérieur et leurs parties hydrophobes à l'Intérieur des bi-couches.

De façon spécifique, les molécules liposolubles peuvent être piégées dans les bi-couches phospholipidiques.

Dans cette voie, les anesthésiques lipophiles sont des candidats pour le piégeage dans les liposomes, de même que peuvent l'être d' autres principes actifs administrables par la voie péridurale tels que des analgésiques centraux du type morphinique.

Parmi ces anesthésiques, il faut citer les anesthésiques locaux choisis parmi les dérivés de l'acide benzoïque, les dérivés de l'anilide ou les dérivés de la cocaïne ainsi que les analgésiques centraux,

morphine ou dérivésde morphine.

Les liposomes mis en oeuvre dans ces compositions sont des liposomes multilamellaires.

La nature des composants entrant dans l'élaboration des liposomes pourra jouer sur la modulation de l'activité des principes actifs encapsulés mais quelle que soit la composition, les effets avantageux qui seront décrits ci-après demeureront pour l'essentiel.

A titre d'exemple, une composition phosphadidylcholine (Pc)/cholestérol (Ch) notamment dans un rapport molaire 4/3 sera particulièrement intéressante.

La préparation des liposomes selon l'invention peut être effectuée selon les procédés connus, le principe actif étant encapsulé de préférence au moment de la formation des liposomes.

Pour ce faire, le principe actif, de préférence sous forme d'une base liposoluble, est mis en solution chloroformique avec les deux composants des liposomes, respectivement le Pc d'oeuf et Ch dans un rapport molaire de 4 : 3.

Les quantités respectives de phospholipides et de principe actif peuvent varier.

Suivant un mode privilégié de réalisation des liposomes, selon la présente invention, on met en solution dans du chloroforme, 60 mg environ de lipides et environ 4 à 5 mg d'anesthésique sous forme de base liposoluble.

Le tout est évaporé sous atmosphère d'azote, puis sous vide pendant une nuit.

La formation des liposomes varie ensuite selon que l'on veut obtenir des MLV ou des SUV. Dans le premier cas, on ajoute environ 3 ml de tampon Tris-HCl 0,3 M à pH 8, au film lipidique obtenu, et on effectue 5 centrifugations dans le même tampon ; dans le second cas, on procède à une sonication pendant environ 15 minutes à 65 W.

Les liposomes avec l'anesthésique ainsi incorporé sont administrés sous forme de compositions pharmaceutiquescontenant en outre un support acceptable pour injection en particulier par voie péridurale.

Les compositions selon la présente invention sont essentiellement utilisables sous forme injectable, c'est-à-dire avec un excipient compatible avec les liposomes et la voie utilisée ; le plus souvent, il pourra s'agir de sérum physiologique.

Les compositions selon l'invention peuvent également comporter d'autres principes actifs que les anesthésiques ou les analgésiques par exemple dans le cas de la bupivacaïne, il est possible d'utiliser en combinaison de l'épinéphrine qui pourra si cela est nécessaire être également encapsulée.

Bien que la voie péridurale soit préférée, d'autres voies sont envisageables suivant le but recherché (anesthésie chirurgicale ou analgésie pure).

Dans ces conditions, les posologies pourront varier selon le poids et l'état du patient ainsi que selon le but poursuivi, ceci sera souvent laissé à l'appréciation de l'anesthésiste du praticien.

De façon surprenante, les expérimentations effectuées avec les compositions selon l'invention ont montré que l'encapsulation de la drogue dans les liposomes conduit à une action anesthésique supérieure par rapport à la drogue non encapsulée, à une plus longue durée d'action à doses égales, à une possibilité de réduire les doses pour obtenir une action similaire et à une diminution des effets secondaires.

Les exemples et figure ci-après sont donnés à titre purement indicatif et permettent d'illustrer d'autres caractéristiques et avantages de la présente invention.

La figure unique représente les résultats concernant la cinétique sanguine après administration de :

- SUV à base de Pc marqué au $C_{14}$ représenté par la courbe 1 (X—–X),
- MLV contenant de la bupivacaïne marquée par $H^3$ représenté par la courbe 2 (⊙— — —⊙),
- bupivacaïne associée à de l'adrénaline représentée par la courbe 3 (⊗—•—•—⊗),
- bupivacaïne seule représentés par la courbe 4 (•— — —•).

EXEMPLE 1: Préparation des liposomes et incorporation de bupivacaïne

(1-1) Préparation des liposomes seuls

Les liposomes, partie essentielle des compositions pharmaceutiques, selon la présente invention, sont préparés par des techniques connues. Il comportent, de préférence deux composés essentiels qui sont : la phosphatidylcholine (Pc) d'oeuf et le cholestérol (Ch) dans un rapport molaire de 4 : 3 et à une concentration totale en lipides d'environ 20 mg/ml.

Selon un procédé préféré de préparation des liposomes selon la présente invention, on mélange la Pc d'oeuf et le Ch dans une solution chloroformique, que l'on évapore sous azote, puis sous vide, pendant une nuit.

Puis les liposomes sont formés de la façon suivante : selon que l'on additionne environ 3 ml de tampon phosphate 0,3 M à un pH d'environ 7,4 ou que l'on procède à une sonication pendant 15 min. à 65 W, l'on

obtient respectivement, soit des liposomes multilamellaires (MLV) soit des liposomes unilamellaires (SUV).

Les liposomes obtenus sont administrés dans l'espace péridural sous forme de compositions pharmaceutiques, contenant en outre un support acceptable, à titre de liposomes de référence.

(1-2) Préparation des liposomes-bupivacaïne

On ajoute à 60 mg de lipides composés de PC d'oeuf et de Ch dans un rapport molaire de 4 : 3, 4,5 mg de bupivacaïne base soluble, et on met le tout en solution dans du chloroforme.

Après évaporation sous atmosphère d'azote et sous-vide pendant une nuit, on forme des liposomes MLV par addition de 3 ml de tampon Tris-HCl 0,3 M par pH8 et par 5 centrifugations à 5000 cpm dans le même tampon.

EXEMPLE 2 : Biodistribution et pharmacocinétique des liposomes ayant incorporé de la bupivacaïne, administrés par voie péridurale

L'étude a porté sur des MLV ayant incorporé de la bupivacaïne, à raison de 4,5 mg de bupivacaïne - base soluble pour 60 mg de lipides comportant du Pc d'oeuf et Ch à raison d'un rapport molaire de 4 : 3 (voir exemple (1-2)).

La bupivacaïne est utilisée en chimie depuis 1963, et possède la formule suivante :

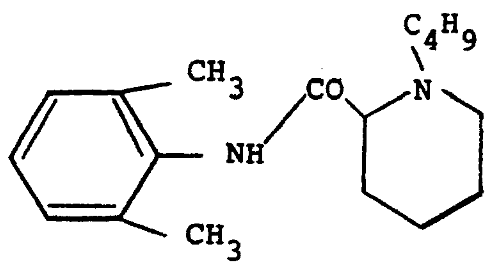

La formation des MLV a lieu par addition de 3 ml de tampon Tris-HCl 0,3 M à pH 8, puis 5 centrifugations à 5 000 G dans le même tampon.

0,2 ml du culot de centrifugation sont administrés par voie péridurale à des lapins, dans les mêmes conditions que dans l'exemple 1.

A titre de contrôle, on administre dans les mêmes conditions 4,5 mg de bupivacaïne à usage clinique (Marcaïne [R] - Astra - Solution chlorhydratée à 5%) avec ou sans adrénaline, additionné de 0,01 mCi de bupivacaïne-$H^3$, le tout dans une solution de 0,2 ml.

De même, à titre de témoin, on administre au cours de cette étude, des SUV sans bupivacaïne, dans les conditions prévues par l'exemple 1.

Le sang des lapins est prélevé 15,30,60, 120,180 et 240 minutes après l'injection.

Les résultats sont exprimés en pourcent de la dose injectée (D.I.) dans le sang total.

Les animaux sont sacrifiés 120 et 240 minutes après l'injection ; on prélève les organes et tissus dans les mêmes conditions que celles de l'exemple 1.

(2-1) Les résultats concernant la cinétique sanguine sont représentés dans la figure.

Les taux sanguins les plus élevés (1,4 % de la dose injectée sont atteints dans les 15 à 60 minutes qui suivent l'injection de bupivacaïne seule).

Quand de l'adrénaline est ajoutée à la bupivacaïne, le pic sanguin est de 0,8 %. Ceci est dû à l'action vasoconstrictive de l'adrénaline réduisant le passage de l'endothélium des capillaires qui traversent l'espace péridural. Sous cet effet, les taux circulants sont même plus faibles que ceux observés après administration de liposomes SUV sans adrénaline.

Pour les liposomes MLV-bupivacaïne, on trouve les taux sanguins les plus bas (± 0,45 %). En outre, on ne note la présence de bupivacaïne dans le sang que 60 minutes après l'injection.

Du point de vue du passage dans la circulation et des toxicités systémiques (cardiaques) qui en sont la conséquence, le liposome MLV-bupivacaïne présente donc une sécurité supérieure à celle des formes de l'anesthésique actuellement utilisées ainsi qu'à celles des liposomes SUV.

(2-2) Les résultats concernant l'excrétion urinaire se trouvent dans le tableau I :

Radioactivité excrétée dans l'urine (D.I.x $10^2$), 2 heures après l'injection péridurale de SUV marquées par Ch-$H^3$, de MLV ayant intégré de la bupivacaïne marquée par $M^3$, ou de bupivacaïne non encapsulée. Moyenne ± écart type ; le nombre entre parenthèses = le nombre d'animaux.

**TABLEAU I**

| SUV Cholestérol-$H^3$ | MLV-bupivacaïne-$H^3$ | bupivacaïne |
|---|---|---|
| 0 | 5,808 ± 0,175 (3) | 11,633 ± 0,518 (3) |

La quantité excrétée de bupivacaïne ou de ses produits de métabolisation est moitié plus faible après injection péridurale de MLV-bupivacaïne que de bupivacaïne. Ceci est à mettre en rapport avec le passage dans la circulation générale et avec les taux sanguins.

(2-3) Les résultats concernant le liquide céphalo-rachidien se trouvent dans le tableau II :
Marquage du liquide céphalo-rachidien (DI dans 1,5 ml x $10^3$) 2 et 4 heures après l'injection péridurale de SUV Ch-$H^3$, MLV ayant incorporé de la bupivacaïne-$H^3$ , ou de bupivacaïne non encapsulée. Moyenne ± écart type ; nombre entre parenthèses = nombre d'animaux.

**TABLEAU II**

| | SUV Cholestérol-$H^3$ | MLV bupivacaïne-$H^3$ | bupivacaïne |
|---|---|---|---|
| 2 heures | 0 (3) | 0 (3) | 0,256 ± 0,018 (3) |
| 4 heures | 0 (3) | 0 (3) | 0,111 ± 0,003 (3) |

On constate une absence totale de marquage après injection péridurale de SUV et de MLV et la présence de molécules radioactives après injection de bupivacaïne ce qui est un phénomène connu, responsable des manifestations toxiques au niveau du système nerveux central. Les liposomes encapsulant l'anesthésique évitent donc cette toxicité.

(2-4) Les résultats concernant les taux hépatiques et cardiaques se trouvent sur le tableau III:
Biodistribution de SUV Ch-$H^3$ , des MLV ayant intégré de la bupivacaïne-$H^3$, et de bupivacaïne, dans le foie et le coeur, 4 heures après l'injection péridurale (D.I. x $10^2$).
Moyenne ± écart type.
Nombre entre parenthèses = nombre d'animaux.

## TABLEAU III

| Organe | SUV Cholestérol-$H^3$ | ~~SUV~~ MLV-Bupivacaïne-$H^3$ | bupivacaïne |
|---|---|---|---|
| foie | 10,353 ± 1,009 (3) | 7,449 ± 1,185 (3) | 20,477 ± 1,458 (3) |
| coeur | 0,605 ± 0,037 (3) | 0,500 ± 0,030 (3) | 2,142 ± 0,301 (3) |

Les taux cardiaques sont plus faibles après injection de MLV que de SUV (en rapport avec les taux circulants). L'accumulation cardiaque de bupivacaïne encapsulée ne représente que 25% de celle observée après injection de bupivacaïne. Ceci est très important pour éviter les risques cardiotoxiques de l'anesthésique.

Les taux hépatiques sont aussi les plus bas après injection de MLV-bupivacaïne (à relier à nouveau au passage dans la circulation).

(2-5) Les résultats concernant la concentration dans les tissus nerveux se trouvent dans le tableau IV. Captage de SUV Ch-$H^3$, de MLV ayant intégré la bupivacaïne $H^3$, et de bupivacaïne, par les tissus nerveux, 2 et 4 heures après l'injection péridurale (D.I dans 1 g x $10^3$) Moyenne ± écart type ; nombre entre parenthèses = nombre d'animaux.

TABLEAU IV

| Tissu | Temps | SUV Cholestérol-$H^3$ | MLV-bupivacaïne-$H^3$ | bupivacaïne |
|---|---|---|---|---|
| racines nerveuses | 2 hrs | NT | NT | NT |
| | 4 hrs | 0,278 ± 0,042 (3) | 2,535 ± 0,145 (3) | 0,153 ± 0,023 (3) |
| moëlle lombaire | 2 hrs | 0,303 ± 0,032 (3) | 1,936 ± 0,130 (3) | 0,411 ± 0,094 (3) |
| | 4 hrs | 0,303 ± 0,031 (3) | 0,718 ± 0,002 (3) | 0,169 ± 0,046 (3) |
| Queue de cheval | 2 hrs | 0,423 ± 0,066 (3) | 1,209 ± 0,187 (3) | 0,485 ± 0,057 (3) |
| | 4 hrs | 0,374 ± 0,072 (3) | 1,806 ± 0,281 (3) | 0,132 ± 0,035 (3) |
| Nerf sciatique | 2 hrs | 0,307 ± 0,035 (3) | 2,143 ± 0,077 (3) | 0,281 ± 0,026 (3) |
| | 4 hrs | 0,500 ± 0,060 (3) | 1,018 ± 0,213 (3) | 0,132 ± 0,028 (3) |
| plexus brachial | 2 hrs | 0,277 ± 0,038 (3) | 1,787 ± 0,049 (3) | 0,295 ± 0,048 (3) |
| | 4 hrs | 0,373 ± 0,032 (3) | 0,775 ± 0,091 (3) | 1,136 ± 0,008 (3) |

Partout, les concentrations les plus fortes se rencontrent pour la bupivacaïne encapsulée dans les MLV, 2 ou 4 heures après l'injection.

L'ordre des concentrations est partout MLV > SUV > bupivacaïne.

Ces résultats soulignent le tropisme élevé des MLV pour les racines nerveuses, le nerf sciatique et le

plexus brachial. Ce qui laisse supposer :

- une action anesthésique supérieure pour la drogue encapsulée ;
- une possibilité de réduire les doses pour obtenir une action similaire ;
- une plus longue durée d'action à doses égales.

De plus, en ce qui concerne à présent les effets observés, un blocage moteur des pattes postérieures a été observé après administration péridurale (niveau S2-S3) de bupivacaïne encapsulée dans des MLV. La bupivacaïne utilisée aujourd'hui en anesthésie bloque les fibres sensibles et non les fibres motrices. Ceci laisse supposer une meilleure pénétration de la drogue encapsulée dans les fibres nerveuses, par rapport à la drogue non encapsulée.

Enfin, concernant la recherche d'une toxicité éventuelle au niveau des fibres nerveuses, une recherche anatomo-pathologique a été effectuée au niveau des racines nerveuses, du sciatique, du plexus brachial, de la queue de cheval et des méninges, 5 heures après l'injection péridurale de MLV. Aucune atteinte n'a été observée au niveau histologique. Ces résultats sont prometteurs, mais il faut noter que des atteintes nerveuses, dans l'emploi des anesthésiques, peuvent apparaître 4 à 6 semaines après l'injection.

## Revendications

1. Composition pharmaceutique, caractérisée en ce qu'elle contient un anesthésique local et/ou un analgésique central, comportant une partie lipophile, encapsulé dans des liposomes essentiellement multilamellaires.

2. Composition pharmaceutique selon la revendication 1, caractérisée en ce qu'elle est sous une forme injectable.

3. Composition pharmaceutique selon l'une des revendications 1 et 2, caractérisée en ce que la composition des liposomes est PC d'oeuf et Ch dans le rapport 4 : 3.

4. Composition pharmaceutique selon l'une des revendications 1 à 3 , caractérisée en ce que l'anesthésique local est un dérivé choisi parmi les dérivés de l'acide benzoïque, les dérivés de l'anilide ou les dérivés de la cocaïne.

5. Composition pharmaceutique selon l'une des revendications 1 à 4 , caractérisée en ce que l'anesthésique est la bupivacaïne.

6. Composition pharmaceutique selon l'une des revendications 1 à 3, caractérisée en ce que l'analgésique est la morphine ou un dérivé de la morphine.

7. Utilisation de liposomes multilamellaires pour la préparation de médicaments à effet anesthésique local et/ou analgésique central encapsulés.

## Claims

1. Pharmaceutical composition, characterized in that it contains a local anesthetic and/or a centrally acting analgesic, comprising a lipophilic portion, which is encapsulated in essentially multilamellar liposomes.

2. Pharmaceutical composition as claimed in claim 1, characterized in that it is in an injectable form.

3. Pharmaceutical composition as claimed in claims 1 and 2, characterized in that the composition of the liposomes is egg phosphatidylcholin and cholesterol in the ratio 4:3.

4. Pharmaceutical composition as claimed in claims 1 to 3, characterized in that the local anesthetic is a derivative selected frcm the benzoic acid derivatives, the anilide derivatives or the cocaine derivatives.

5. Pharmaceutical composition as claimed in claims 1 to 4, characterized in that the anesthetic is bupivacaine.

6. Pharmaceutical composition as claimed in claims 1 to 3, characterized in that the analgesic is the morphine or a morphine derivative.

7. Use of the mulilamellar liposomes for the preparation of encapsulated drugs with local anesthetic and/or a centrally acting analgesic effect.

**Patentansprüche**

1. Pharmazeutische Zusammensetzung, **dadurch gekennzeichnet, daß** sie ein lokales Anästhetikum und/oder ein zentrales Analgetikum enthält, die einen lipophilen Teil aufweisen und in im wesentlichen multilamellaren Liposomen eingekapselt sind.

2. Pharmazeutische Zusammensetzung gemäß Anspruch 1, **dadurch gekennzeichnet, daß** sie in injizierbarer Form vorliegt.

3. Pharmazeutische Zusammensetzung gemäß einem der Ansprüche 1 oder 2, **dadurch gekennzeichnet, daß** die Liposomen aus Ei-Pc und Ch im Verhältnis 4 : 3 bestehen.

4. Pharmazeutische Zusammensetzung gemäß einem der Ansprüche 1 bis 3, **dadurch gekennzeichnet, daß** das lokale Anästhetikum ein Derivat ist, das unter den Derivaten der Benzoesäure, den Derivaten des Anilids oder den Derivaten des Kokains ausgewählt wird.

5. Pharmazeutische Zusammensetzung gemäß einem der Ansprüche 1 bis 4, **dadurch gekennzeichnet, daß** das Anästhetikum Bupivacain ist.

6. Pharmazeutische Zusammensetzung gemäß einem der Ansprüche 1 bis 3, **dadurch gekennzeichnet, daß** das Analgetikum Morphin oder ein Derivat des Morphins ist.

7. Verwendung von multilamellaren Liposomen zur Herstellung von eingekapselten Medikamenten mit lokaler anästhetischer und/oder zentraler analgetischer Wirkung.

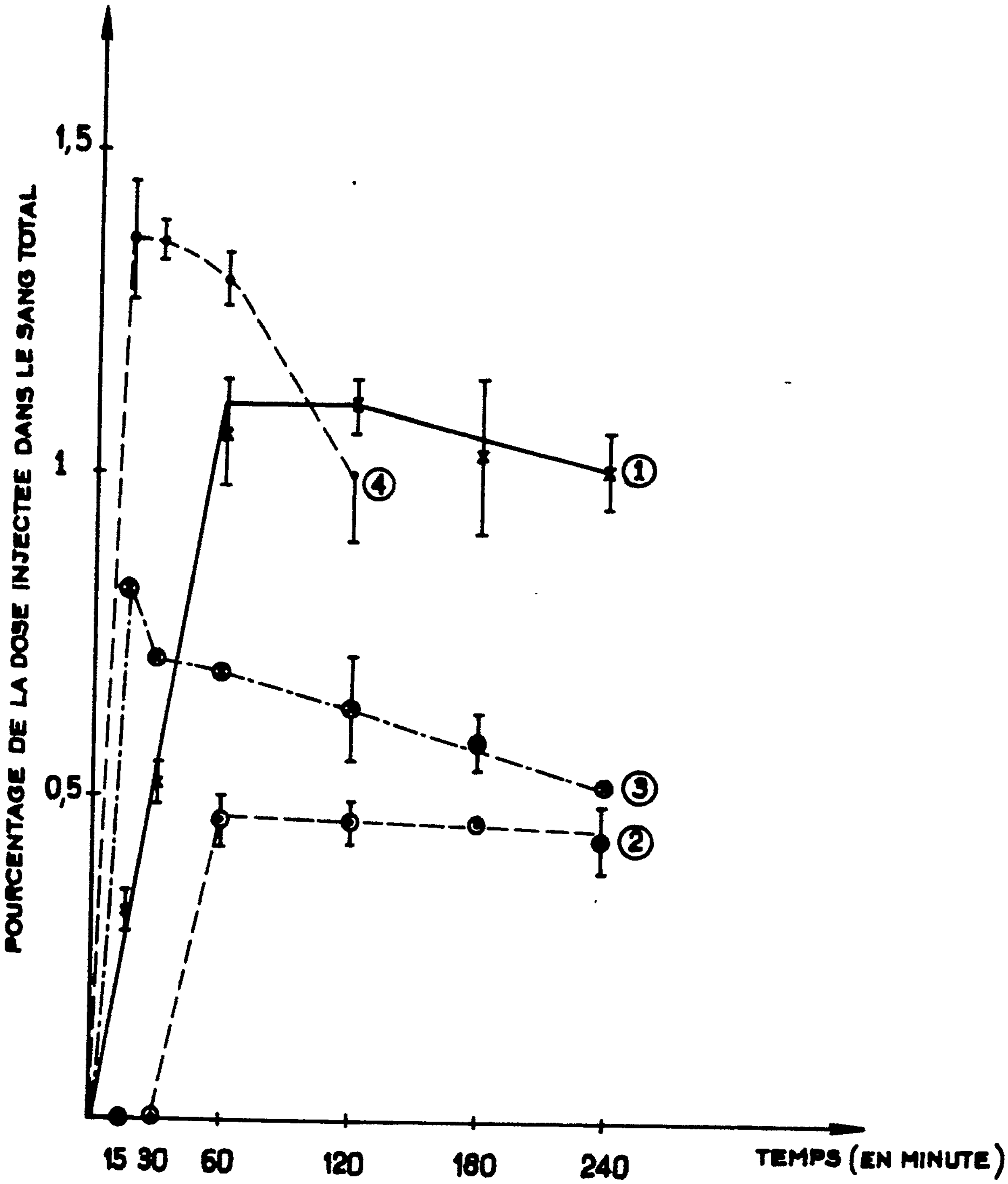
POURCENTAGE DE LA DOSE INJECTEE DANS LE SANG TOTAL
1,5
1
0,5
15
30
60
120
180
240
TEMPS (EN MINUTE)
①
②
③
④